# EUROPEAN PATENT SPECIFICATION

(11) **EP 2 490 736 B1**
(45) Date of publication and mention of the grant of the patent: **22.11.2017**
(21) Application number: 10779349.9
(22) Date of filing: 20.10.2010
(51) Int. Cl.: A61M 15/00

(54) **DRY POWDER INHALER**
TROCKENPULVERINHALATOR
INHALATEUR DE POUDRE SÈCHE

(30) Priority: 20.10.2009 TR 200907917
(43) Date of publication of application: 29.08.2012
(73) Proprietor: Sima Patent ve Lisanslama Hizmetleri Ltd.Sti., Esenler/Istanbul (TR)
(72) Inventor: Bilgic, Mahmut, Istanbul, 34173 (TR)
(86) International application number: PCT/TR2010/000210
(87) International publication number: WO 2011/049541

(56) References cited:
- EP-A1- 2 082 759
- EP-A1- 2 082 771
- WO-A1-2007/012871
- US-A1- 2007 267 016

## Description

### Technical field

The present invention relates to a dry powder inhaler which is used for administering a dry powder medicament via inhalation. The dry powder inhaler in accordance with the present invention is designed for providing easier and more reliable use than the dry powder inhalers which are present in the state of the art.

### The prior art

Since the beginning of the human history, symptoms and diseases, associated with respiratory system, are problems which people has frequently encountered. Enviromental conditions which are getting worse day by day, acquired bad habits, genetic factors increase the probabilty of encountering these problems.

Active agent and/or active agents are administered via inhalation route in the treatment of several respiratory disorders, especially asthma and chronic obstructive pulmonary diseases. Various inhalation devices are designed for delivery of these active agents in sufficient amount to the patient. Said inhalation devices are different from one another because of their mechanisms and components.

The active agents, which are administered for the treatment of the diseases given hereinbefore, can be inhaled either in liquid form or in dry powder form. Because of the certain drawbacks (stability, degradation in a short time, uncontrolled dosing, etc.) arised from the medicaments that are in liquid form, it is preferable that the medicaments used in inhalation therapy, is administered in dry powder form.

There are lots of inhalation devices used for delivery of the dry powder medicament. Depending on the properties and the working mechanism of the inhalation device, the dry powder medicament can be carried in a reservoir, a capsule or a multi-dose blister pack.

One dose of the medicament in the reservoir is prepared for the inhalation with each actuation of the inhalation device. However, due to the fact that the dry powder medicament is not covered by an extra package, the stability of the dry powder medicament can not be maintained for a long time. It is difficult to provide delivery of an accurate dose of the medicament by using this type of inhalation device. In addition to this, the amount of medicament in the reservoir, which is considerably little, and difficulty to use this type of inhalation device, causes the drawbacks in the use of this inhalation device by the patients.

In use of the inhalation devices which are the single-dose inhalation devices and are used for administering the dry powder medicament from a capsule, the capsule is needed to be perforated before inhaling the medicament. The dry powder medicament which is present in the capsule, is inhaled from the capsule by passing through one or more holes made in the course of perforation of the capsule. Therefore, some medicament which is not inhaled by the patient, remains in the perforated capsule after the inhalation. Because of the fact that the hygiene is not provided sufficiently while the capsule is being loaded to the capsule chamber of the inhalation device for each inhalation, there is a risk of the inhalation of the foreign matter together with the dry powder medicament. Additionally, several additional mechanic components are needed for the perforation of the capsule. These additional mechanical components lead to increase in the cost and size of the inhalation device. On the other hand, because of the fact that the deformation of the needles which perforate the capsule, the capsule can not be perforated properly and the dry powder medicament which is present in the capsule can not be inhaled effectively. So that, the sufficient amount of the dry powder medicament is not delivered to the target area of the patient.

The multi-dose inhaltion devices are designed for eliminating the drawbacks which are encountered in the course of the inhalation of the dry powder formulation from the reservoir or the capsule in the inhalation device. In these inhalation devices, the dry powder medicament is generally inhaled from a pack in which cavities, each of which contains one dose of the dry powder medicament, are juxtaposed. In each inhalation, one dose of the medicament is ready for inhalation as a result of peeling or perforating of said pack. This type of devices are intended to be reliable and used for a long time.

The inhalation device which is sold by GlaxoSmithKlein under the tradename Diskus®, is one of the well-known multi-dose inhalation devices in the market. In this inhalation device, the dry powder medicament is carried in the cavities which are juxtaposed along the blister strip. The mouthpiece and the finger tab appear by moving the mouthpiece cover to its open position. The lever associated with the finger tab, actuates the gear mechanism as the finger tab is slided from end to end. Therefore, the blister strip is advanced, one blister is opened, and one dose of the dry powder medicament contained in the blister cavity, become ready for the inhalation. A pawl engages the teeth of the base winding wheel to prevent the wheel moving anticlockwise, thus ensuring that the blister strip can only proceed forwards through the device.

Despite of the efficacy of Diskus® in the inhalation therapy, multiple operations are needed to applied in use of this inhalation device for making the dry powder formulation ready for inhalation.

WO 2007/012871, WO 2007/068900, EP2082771 or WO 2005/014089 describes an inhalation device comprising a mouthpiece cover which is movably mounted to the inhalation device for sequential movement from a first position, to a second position and then to a third position. When mouthpiece cover is moved from the first position to the second position, the movement does not actuate the dispensing mechanism and hence no blister opens. So that, the moutpiece can be cleaned without actuation of the inhalation device. The movement of the mouthpiece cover from the second position to the third position results in actuation of the dispensing mechanism and making at least one blister open.

WO 2005/037353 describes an inhalation device in which the moutpiece cover is needed to be moved at least 90° to couple with the dispensing mechanism of the inhalation device. However, said inhalation devices in which there is a risk of accidentally moving the moutpiece cover from the a position, in which the mouthpiece is covered and it can be cleaned, to the another position, in which the mouthpiece is uncovered and the dispensing mechanism has been actuated. Therefore, despite of the fact that it is not an inhalation time, the inhalation device is actuated and one dose of the dry powder medicament is wasted.

Furthermore, WO 2007/012871 or WO 2007/068900 describes an inhalation device in which the cover interacts with a ratchet which is provided with an irreversible feature to prevent reverse rotation thereof and hence to prevent reverse rotation of the blister strip(s). The movement of the moutpiece cover cause the resilient pawl legs of the ratchet mechanism to engage the inner teeth of the ratchet gear. After said resilient pawl legs engage the inner teeth of the ratchet gear, if the mouthpiece cover is kept on moving through the inhalation device, the ratchet gear actuates the dispensing mechanism and hence the blister strip(s) advance. The advancement of the blister strip(s) leads to actuation of the dose counter which counts the number of doses left to be taken or the number of doses taken. In addition to this, the ratchet mechanism comprises complicated mechanical components to provide an irreversible rotation of the blister strip(s) and to make the dispensing mechanism work properly. Consequently, to have these mechanical components increases the cost, size and complexity of the inhalation device as well as affecting negatively user friendliness of the inhalation device.

Because of all the drawbacks given above, there is a need to devise a dry powder inhaler which is used for administering the dry powder medicament from the blister cavity in a reliable and easy way.

The present invention provides a dry powder inhaler which is cost-effective, reliable, easy and hygienic to use and delivers an accurate dose of the dry powder medicament to the lung to meet the requirements given above completely for achieving an effective inhalation.

### Summary of the invention

Described is a dry powder inhaler comprises:
- a mouthpiece through which the dry powder medicament is inhaled by the patient,
- a mouthpiece cover for covering said mouthpiece,
- an elongate, peelable blister strip containing the dry powder medicament,
- a dispensing mechanism which is actuated by the movement of mouthpiece cover to open one blister of the blister strip and making it ready for inhaling the dry powder medicament from said blister,
- a stopper
wherein before the inhalation of dry powder medicament, only a single movement is applied which is the movement of the mouthpiece cover from one position in which the mouthpiece is completely covered to another position in which:
- the mouthpiece is completely uncovered,
- said stopper engages to a component of said dry powder inhaler for preventing the reverse rotation of the peelable blister strip, and
- said dispensing mechanism is actuated for advancing and opening one blister, making the medicament ready to be inhaled.

The dry powder which is devised to achieve an effective inhalation, consists of the mechanical components. Said dry powder inhaler comprises a housing on which the mechanical components which are required to achieve an effective inhalation of the dry powder medicament, are located. These mechanical components constitute the dispensing mechanism which is actuated as a result of the movement of the mouthpiece cover.

According to the invention, the dispensing mechanism which causes the advancement of the blister strip, interacts with directly to the moutpiece cover through the center of the dry powder inhaler and comprises the gear mechanism of the dry powder inhaler. The dispensing mechanism is actuated by the fact that the movement of the mouthpiece cover is transmitted to the dispensing mechanism via a central hub gear of the dispensing mechanism. So that, before the inhalation of the dry powder inhaler, the single movement which is the movement of the mouthpiece cover from one position to another position, results in that the mouthpiece is completely uncovered, the peelable blister strip is advanced by working of the dispensing mechanism for inhaling the dry powder medicament from the blister and the dose counter shows the number of doses left to be taken. Therefore, the patients from every age, can use the dry powder inhaler in accordance with the present invention easily and inhale the dry powder medicament fastly and effectively.

According to the invention, the indexing / advancement of the peelable blister strip is achieved by a rotatable index wheel. This rotatable index wheel having recesses therein, is engageable with the peelable blister strip in use with said dry powder inhaler such that said recesses each receive a respective cavity of the base sheet of a blister strip in use with said dry powder inhaler. The index gear interacting with the index wheel can be provided with a stopper or a lock shaft to provide irreversible rotation of the peelable blister strip. Because of the fact that the stopper engages the index gear interacting with the index wheel after the advancement of the peelable blister strip, the peelable blister strip is present at a certain position wherein one blister is opened completely and the dry powder medicament in this blister can be inhaled by the patient. In each actuation of the dispensing mechanism, the index wheel rotates irreversibly with the same angle, so that, open blister of the blister strip is situated in correct and accurate position for inhaling the effective amount of the dry powder medicament from open blister with high discharging capacity. The stopper can attach to the top cover of said dry powder inhaler to engage the movable mouthpiece cover for providing irreversible movement of said mouthpiece cover as well as it also engages the index gear interacting with the index wheel after the advancement of the peelable blister strip. In each actuation of the dispensing mechanism, the movement of the mouthpiece cover from one position, in which the mouthpiece is completely uncovered, to another position, in which the mouthpiece is completely closed, results in the advancement of the peelable blister strip to the same extent. The stopper which engages the movable mouthpiece cover to prevent its reversible movement from the position in which the mouthpiece is completely uncovered and open blister of the peelable blister is situated correctly for an effective inhalation of the dry powder medicament from opened blister. Also, the stopper which is attached to the top cover of said dry powder inhaler, make it possible to prevent reversible rotation of the index wheel undirectly because of the fact that the movable mouthpiece cover is attached directly to the dispensing mechanism through the center of the dry powder inhaler.

According to the present invention, the dry powder inhaler in which the index gear and the other gears interacting with the index gear directly or indirectly engage with one another correctly so that the dispensing mechanism works properly for delivering effective amount of the dry powder medicament to the lungs in each inhalation. It is important that the engagement especially between the gear of the index wheel and central hub gear is proper and tight because of the fact that the movement of the mouthpiece cover, which causes the actuation of the dispensing mechanism, is transmitted to the index gear in the dispensing mechanism via central hub gear.

The peelable blister strip, which consists of the blisters wherein each of the blisters has one dose of the dry powder medicament, is hold as winding up around itself in the blister strip chamber of the dry powder inhaler and is in elongate form. A base sheet and lid sheet of the peelable blister strip are peeled apart from each other to open a blister for inhaling the dry powder medicament contained by it. The actuation of the dispensing mechanism as a result of the movement of the mouthpiece cover leads to advancement of the blister. While the peelable blister strip is advanced by actuating the dispensing mechanism, the base sheet and the lid sheet of the peelable blister strip are peeled apart from each other to open one blister and the dry powder medicament in open blister become ready for inhalation in the opening station. The base sheet and the lid sheet of the peelable blister, which are peeled apart from each other while the peelable blister strip is advanced, gather in separate parts of the inhaler. The lid take-up mechanism comprises a wheel around which the lid sheet is wound tightly and it uses torsional force on the lid sheet for pulling apart the lid sheet from the base sheet of the blister that has been received at the opening station in which the dry powder medicament is inhaled from open blister. The base sheet, in which blisters are formed to define blister pockets (cavities) therein for containing distinct medicament dose portions, is wound around the base sheet take-up spindle which is hold in another part of the housing.

Another aspect of the invention, the moutpiece cover is present in only two positions: the first position in which the mouthpiece is completely covered for protection of this mouthpiece, the second position in which the mouthpiece is completely uncovered, the mouthpiece can be cleaned easily and the dry powder medicament can be inhaled through the mouthpiece.

In opening station of the dry powder inhaler, there is a beak, and the lid sheet and the base sheet are separable by peeling to open a blister cavity about this beak. After the blister cavities passes through the opening station, because of the engagement between recesses of the index wheel and blister cavities, the blister strip, which has been advanced as a result of the actuation of the dispensing mechanism, is positioned correctly and accurately in the dispensing station for achieving the effective inhalation of the dry powder formulation. An airflow, which enters to the dry powder inhaler throughout at least an airflow inlet during the inhalation of the patient through the dry powder inhaler, reaches to the dry powder medicament in the cavity of open blister in the dispensing station. Said dry powder medicament is drawn out through manifold of the dry powder inhaler and then it is delivered to the patient by the airflow. The airflow inlet can be in any suitable shape which provide for entering of airflow to the dry powder inhaler easily. The manifold through which the dry powder medicament is drawn out by the airflow, can be in any suitable form which enables delivery of the dry powder medicament to the patient in an effective way.

The dry powder has a holder on the movable mouthpiece cover. This holder can be in any suitable form which makes the patients move the mouthpiece cover easily. Therefore, the mouthpiece cover can be moved easily by holding this holder on the mouthpiece cover.

All the mechanical components of the dispensing mechanism is located in the housing which can be in any suitable shape, preferably circle or oval, to provide for working of these mechanical components properly and for interaction of the mechanical components with one another properly.

Various known techniques can be employed to join the lid and base sheet and hence to seal the blisters of the peelable blister strip. Such methods include adhesive bonding, hot metal bonding, hot metal welding, radio frequency welding, laser welding, ultrasonic welding and hot bar sealing. The lid sheet and base sheet of the peelable blister strip are particularly sealable by 'cold form' sealing methods, which are conducted at lower temperatures than conventional heat sealing methods. Such 'cold form' sealing methods are of particular utility where the medicament or medicament formulation for containment within the blister is heat sensitive.

The lid sheet and the base sheet of the peelable blister strip, consists of many layers which constitute the lid sheet and the base sheet of the blister strip such as polymeric layer, aluminum foil and optionally Aclar® fluoropolimer film.

Aclar® fluoropolymer film is a polymeric film which is used for production of the blister strip and provides high moisture protection. This chemically inert film does not cause any change in taste of the formulation when it is in contact with the dry powder formulation. It easily forms a lamellar structure with other polymeric layers which are made from various polymers. It is suitable for treatment with heat.

Desiccant agents are optionally added to the polymeric layers in order to reduce moisture and gas permeability of the polymeric layers for protection of stability of the dry powder formulation contained in the blisters which are juxtaposed on the peelable blister strip. Some examples of the desiccant agents are silica gel, zeolite, alumina, baucsite, anhydrous calcium sulfate, activated carbon, clay capable of absorbing water. Aluminium foil is used both in the lid sheet and in the base sheet of the peelable blister strip to provide high humidity and gas protection because of that aluminium foil is conventionally used in both the lid sheet and the base sheet of the blister strip for high humidty and gas protection. These layers must have the sufficient thickness which provides the protection for the stability of humidity sensitive dry powder formulation which is carried in the blister cavity. Because of this reason, the thickness of aluminium foil that is used in the lid sheet and the base sheet of the blister strip is in the range of 10 to 40 µm, preferably of 15 to 30 µm.

The polymeric layers which are contained in the lid sheet and the base sheet of the peelable blister strip in accordance with the present invention may be made from either same or different polymers. The thickness-of these polymeric layers depends on the type of polymeric substance used and its properties. Therefore, the thickness of each polymeric layer which is used in the lid sheet and the base sheet of said blister strip is in the range of 15 to 60 µm, preferably of 20 to 35 µm depending on the type of polymer used.

The inside layer of blister cavity of said blister strip which is in contact with dry powder formulation is polymeric layer because of the fact that aluminium foil causes adhesion of a part of dry powder formulation to inside layer of the cavity due to electrostatic forces, and hence cause uncontrolled dosing. The polymers used for forming polymeric layers are preferably selected from a group comprising thermo-plastic polymers such as polyethylene, polypropylene, polystyrene, polyolefin, polyamide, polyvinyl chloride, polyurethane, or synthetic polymers.

Moreover, said blisters which are placed on the peelable blister strips mentioned above, can be in any appropriate shape. Blisters that are placed to the bottom sheet of the blister strip can be in the same or different shapes and volumes and depending on the type of treatment can comprise dry powder medicament in the same or different amounts.

Said dry powder form, which is carried in the blister cavities of the peelable blister strip is produced with methods known in the prior art. The medicament formulation which is in dry powder form, contains the active agents with the particle size of less than 20 µm. Preferably, lactose is used as excipient. The excipients with fine and coarser particles which have different particle size range, are used in order to provide effective inhalation of the medicament formulation.

Inhalation device is designed for effective inhalation of the medicament in dry powder form both in monotheraphy wherein medicament in dry powder form comprises a single active agent and in combined theraphy wherein medicament in dry powder form comprises more than one active agent. Moreover, said inhaler device has been designed in a way that medicament in dry powder form that will be used for monotheraphy and combined theraphy will be present in a single blister strip.

Inhalation device can be made up of plastic material that is obtained by injection moulding or another method and any component of the device can be in a color which would provide ease of use. All of the components of the inhalation device can be made of the same or different type of plastic materials. These plastic materials can be selected from a group comprising fluoropolymer, polyethylene, polypropylene, acrylonitrile butadiene styrene, poycarbonate, polyamides, polystyrene or polyurethane.

Figures shown below are given to demonstrate the working mechanism of the inhaler and to show the components of the inhaler clearly, the properties of the inhaler should not b limited with these.

### Brief description of the figures

Figure 1, shows a perspective view of the dry powder inhaler wherein the moutpiece cover is in covered position;
Figure 2, shows a perspective view of the dry powder inhaler shown in Figure 1 in a position in which the mouthpiece is completely uncovered;
Figure 3, shows a perspective view of the movable mouthpiece cover of the dry powder inhaler;
Figure 4, shows a perspective view of the components of the dry powder inhaler which interact with each other;
Figure 5, shows perspective view of the top cover and the bottom cover of the dry powder inhaler;
Figure 6, shows a perspective view of the dispensing mechanism when the lid sheet and base sheet is peeled apart from each other;
Figure 7, shows an exploded horizontal-view of all the components of the dry powder inhalation;
Figure 8, shows a perspective view of index mechanism of the dry powder inhaler;
Figure 9, shows a perspective view of the lid take-up mechanism of the dry powder inhaler
Figure 10, shows a perspective view of the counter mechanism of the dry powder inhaler;
Figure 11, shows an exploded top- view of all the components of the dry powder inhaler;
Figure 12, shows a view of track of the airflow which enter the dry powder inhaler during inhalation;
Figure 13, shows a perpective view of the blister strip of the dry powder inhaler.

### Detailed description of the figures

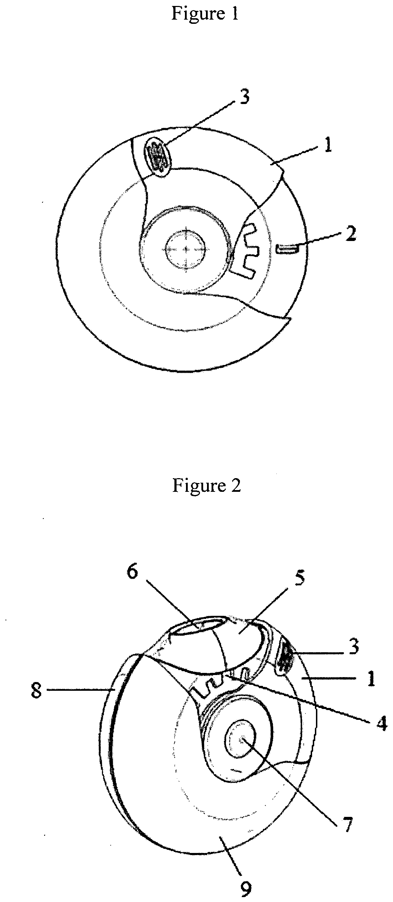

Figure 1 shows movable mouthpiece cover (1) of the inhaler of the present invention in closed state. Holder (3) that is placed on the mouthpiece cover (1) enables patient to act fast in a state of emergency and easily open the mouthpiece(1). The component that is responsible from fixing the movable mouthpiece cover at an accurate position, when it is opened to inhale the medicaments, is the stopper (2). Stopper (2) is beneath the movable mouthpiece cover (1) when mouthpiece (5) is in the uncovered position. The stopper component (2) used in the inhaler, fix the movable mouthpiece cover (1) at the correct and accurate position and while doing this it cause delivery of the medicament in dry powder form by a series of actions comprising actuation of the dispensing mechanism that is attached to the movable mouthpiece cover (1) which leads to indexing of the index wheel (11) with an equal angle upon each opening of the blister strip (20) and therefore fixing the opened blister that is on the recess (11a) of the index wheel (11) at a correct and accurate position and afterwards leading to inhalation of the medicament in dry powder form.

Figure 2 shows inhaler device of the present invention when the mouthpiece (5) is in uncovered position. The movable mouthpiece cover (1) of the inhalation device in accordance with the present invention is present in only two positions. The movable mouthpiece cover (1) is present in a position in which the mouthpiece (5) is completely covered and the movable mouthpiece cover (1) is completely closed in Figure 1. The movable mouthpiece cover (1) is also in the another position in which the mouthpiece (5) is completely uncovered and the mouthpiece cover (1) is completely opened in Figure 2. The movable mouthpiece cover (1) is joined to the dispensing mechanism of the dry powder inhaler through the center of the dry powder inhaler. The movable mouthpiece cover (1) is joined to the bottom cover (8) and the top cover (9) by junction points (7a, 7b). Opening of the mouthpiece cover (1) results in the opening of the airflow inlet and the dry powder inhaler which is ready for the inhalation. Upon inhalation of the patient, the airflow enters the dry powder inhaler through airflow inlet (4) and draw out the dry powder medicament from the opened blister (20a) to the mouthpiece by passing through the manifold (6) to deliver the dry powder medicament to the patient.

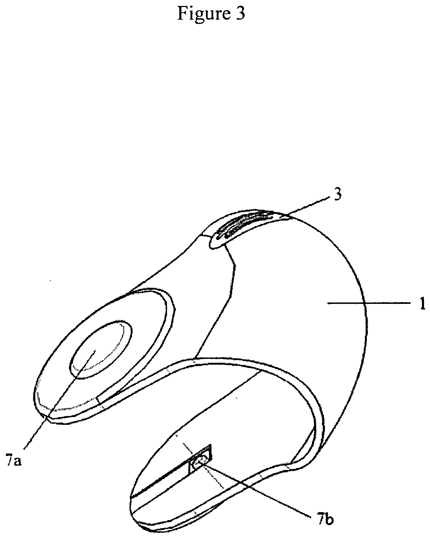

Said junction points (7a, 7b) are showed clearly in Figure 3. The movable mouthpiece cover (1) is joined to the top cover (9) by top junction point (7a) and it is joined to the bottom cover (8) by the bottom junction point (7b).

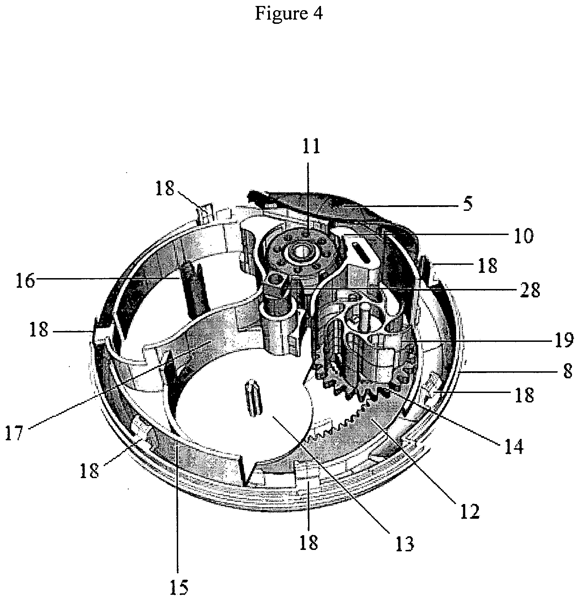

Figure 4 shows a view in which the components of the dispensing mechanism is placed at the housing (15). At the bottom of the dry powder inhaler, there is a chamber (13) in which the blister strip (20) is hold. Said blister strip (20) which is an elongated peelable blister strip, is placed in this chamber (13) in rolled form. The central hub gear (28) interacting with the movable mouthpiece cover (1) is actuated by the movement of the mouthpiece cover (1) and causes the rotation of the gear of index wheel (29), counter gear (12) and lid take-up wheel gear (14). The fact that the lid take up wheel (19) and the lid take up wheel gear (14) rotate together, causes the lid sheet (21) of the blister strip to be wound up around the lid take-up wheel (19) tightly. The base sheet (22) and lid sheet (21) of the blister strip (20) are peeled apart from each other by a beak (10) to open a blister cavity (20a) as the blister cavities (20a) engage the recesses of the index wheel (11a). At the same time, as the lid sheet (21) is wound up around the lid take-up wheel (19) tightly, the base sheet (22) in which the empty blisters are placed, is wound up the shaft (16) in the housing (15). The separator (17) is used in the housing (15) to separate the different parts of the housing (15) in which the components of the dispensing mechanism are placed. There are eight hooks (18) that are arranged one after another with same distance and join the bottom cover (8) and the top cover (9) to each other tightly. The mouthpiece (5) is used by the patient to inhale the dry powder medicement in the opened blister cavity (20a) during the inhalation.

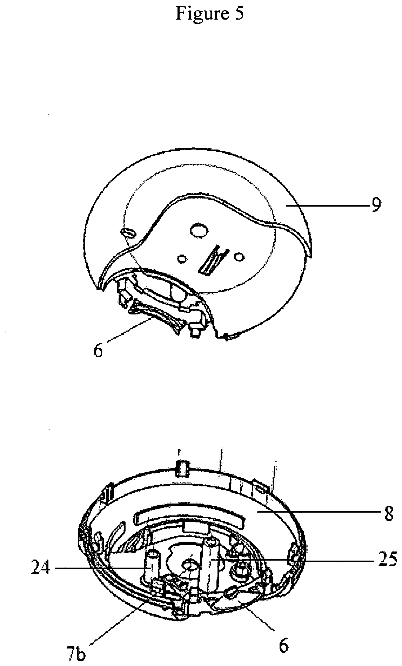

Figure 5 shows a view in which the bottom cover (8) and the top cover (9) are seperate from each other. The mouthpiece cover (1) is not showed in the Figure 5. The shaft (24) at the center of the lid take up wheel gear (27) and the another shaft (25) at the center of the index wheel (11) are placed in the bottom cover (8). Additionally, the manifold (6) through which the dry powder medicament is passed, is placed at the bottom cover (8) of the dry powder inhaler.

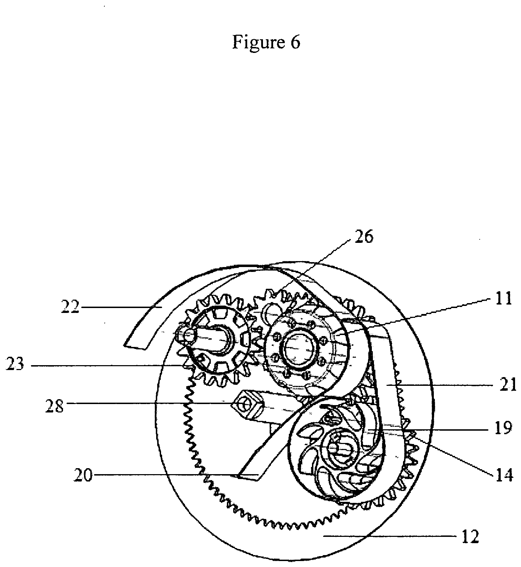

Figure 6, shows the dispensing mechanism when the lid sheet (21) and base sheet (22) of the elongate, peelable blister strip (20) is peeled apart from each other to open a blister cavity (20a) for making the dry powder medicament in it ready for the inhalation. The central hub gear (28) interacting with the movable mouthpiece cover (1) is actuated by the mouthpiece cover (1), in other words, it is rotated by the movement of the mouthpiece cover (1) and this movement of the mouthpiece cover (1) is transmitted to the gear of the index wheel (11) and the lid take up wheel gear (14) via the central hub gear. Upon the rotation of the index wheel (14) as a result of the transmittance of the movement of the mouthpiece cover (1), the blister strip (20) is advanced and the lid sheet (21) is peeled away from the base sheet (22) by the help of the beak (10) to open a blister cavity (20a) in which there is one dose dry powder medicament. The lid sheet (21) has been peeled away from the base sheet (22) before it comes to the dispensing mechanism which consists of the interior component of the dry powder inhaler. The lid sheet (21) of the elongate blister strip (20) is wound up around the lid take-up wheel (19) which moves with the lid take-up wheel gear (19). The base take up spindle gear (23) also causes the counter gear (12) to be rotated. Therefore, the counter gear, on which there is a dose counter (32) that shows doses left to be taken out of the dry powder inhaler, is rotated by the movement of the mouthpiece cover for each actuation of the dispensing mechanism.

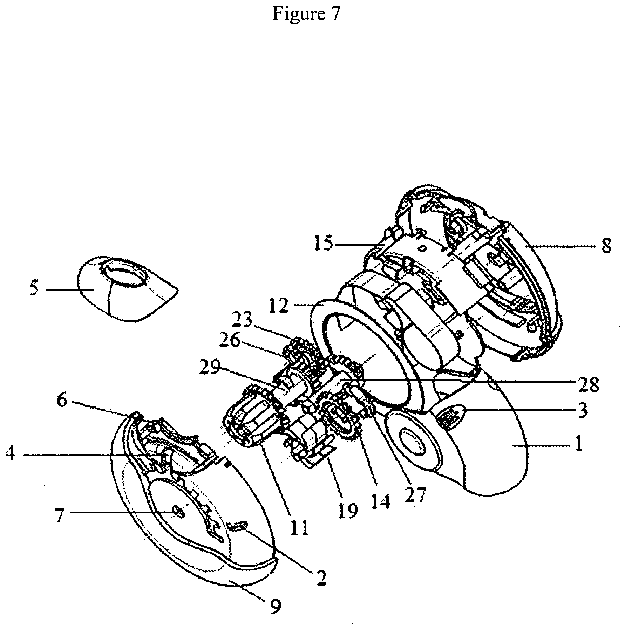

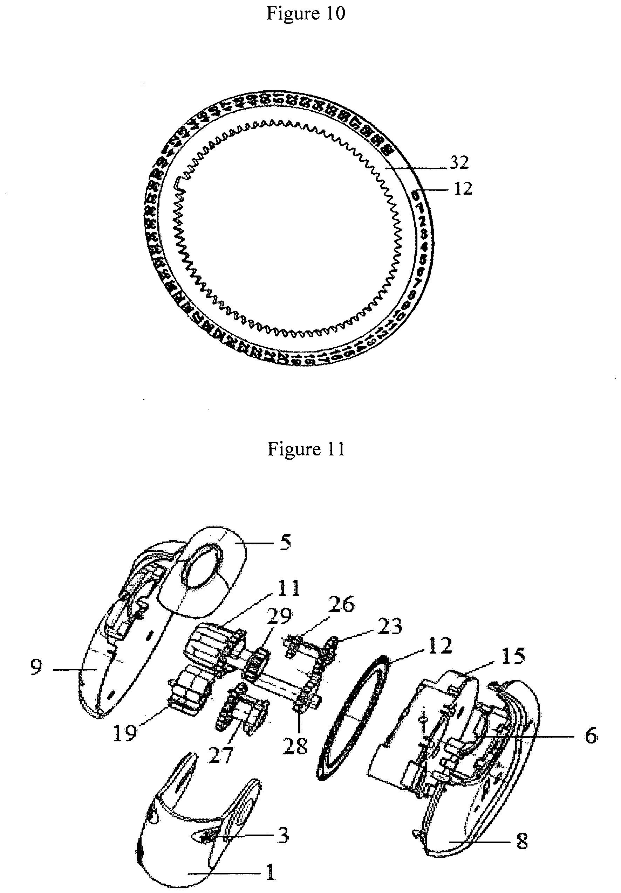

Figure 7 and Figure 11, show exploded views of all the components of the inhaler device from different perspectives. The mouthpiece (5) appears as a result of the movement of the mouthpiece cover (1) by the help of holder (3) before inhalation. Furthermore, the movement of the mouthpiece cover (1) causes the movement of the central-hub gear (28) which joins with the mouthpiece cover (1). Because of the fact that the movement of the mouthpiece cover (1) causes the movement of the central-hub gear (28), the dispensing mechanism of the dry powder inhaler is actuated. So that, the gear of the index wheel (14), idler gear (26), base take up spindle gear (23) and lid take-up wheel gear (14), which engaged each other directly or indirectly, rotate to advance the peelable blister strip (20).

The peelable blister strip (20) is intended to advance in an irreversible rotation and open blister cavity (20a) is intended to be in a correct and accurate position for achieving an effective inhalation. For this purpose, additional mechanical components are used. The stopper (2) is used for providing the irreversible rotation of the blister strip (20) by preventing reversible movement of the mouthpiece cover (1) or by preventing reversible rotation of the index wheel (11), each of them interacts with the blister strip (20). According to figure 1, the stopper (2) is attached to the top cover (9) of the dry powder inhaler. The stopper (2) which engages the movable mouthpiece cover (1) to prevent its reversible movement from the position in which the mouthpiece (5) is completely uncovered and open blister cavity (20a) of the peelable blister (20) is situated correctly for an effective inhalation of the dry powder medicament. For each movement of the mouthpiece cover (1), the stopper (2) fixes the movable mouthpiece cover (1) in an accurate position and provides the mouthpiece cover (1) to move with same angle. This results in that the index wheel (11) rotates with same angle for each actuation of the dispensing mechanism, which is actuated by the movement of the movable (1), and the recess of the index wheel (11a) in which open blister is situated which stops at the correct and accurate position.

The fact that the stopper (2) is engaged to the index wheel (11) to prevent reversible rotation of the index wheel (11) and to provide of positioning of the index wheel (11) in the accurate position leads to achivement of an effective inhalation.

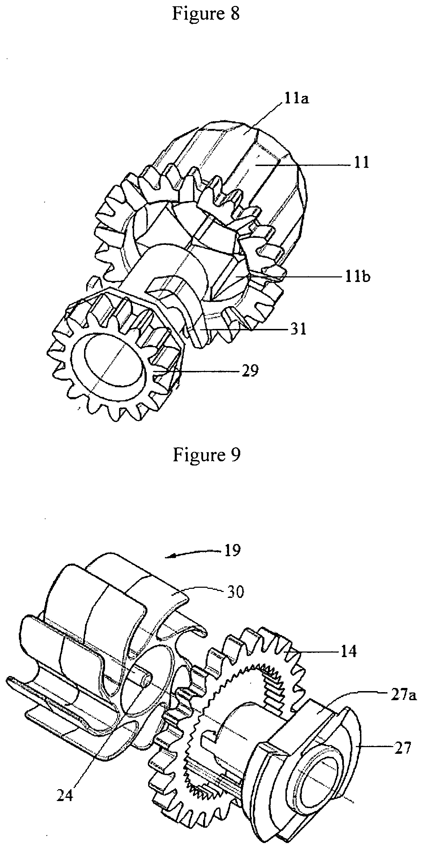

Figure 8 shows the interaction between the index wheel (11) and index wheel gear (29). Legs of the index wheel gear (31) is engaged to the gears which are in the inside of the index wheel (11) to move the index wheel (11). The recesses of the index wheel (11a) are in a suitable shape which enables the blister strip (20) to situate in it easily.

Figure 9 shows the lid take-up wheel and components that interact with it which enables tightly winding up of the lid sheet (21) of the blister strip (20). For proper movement of the blister strip (20) in one way it is curcial to wind-up the lid sheet (21) tightly in an irreversible way. Leg of lid take-up spindle gear (27a) that are present on lid take up spindle gear (27) engage in the gear that are inside the lid take-up wheel gear (14) and lead to one way movement of the lid take-up wheel (19) that is interacting with the lid take-up wheel gear (14). Additionally, resilient legs (30) of the lid take-up wheel (19) flexes to maintain a fixed radius as the lid sheet (21) of the blister strip (20) is wound up. In addition to this, it causes stretching of the lid sheet (21) that is peeled apart from the base sheet (22) of the blister strip (20) and this way leads to proper advancement of the blister strip (20).

Figure 10 shows counter gear (12). The movement of the mouthpiece cover (1) is transferred by central hub gear (28) to another gears of the dispensing mechanism which interact with each other directly or indirectly. One of the gears of the dispensing mechanism, wherein the movement is transferred by central hub gear (28), is counter gear (12). This gear moves according to stoping of the index wheel (11) in dispensing station (42) and by the help of the numarator (32) that has numbers on it, helps the user to understand how many doses left to be taken out of the dry powder inhaler.

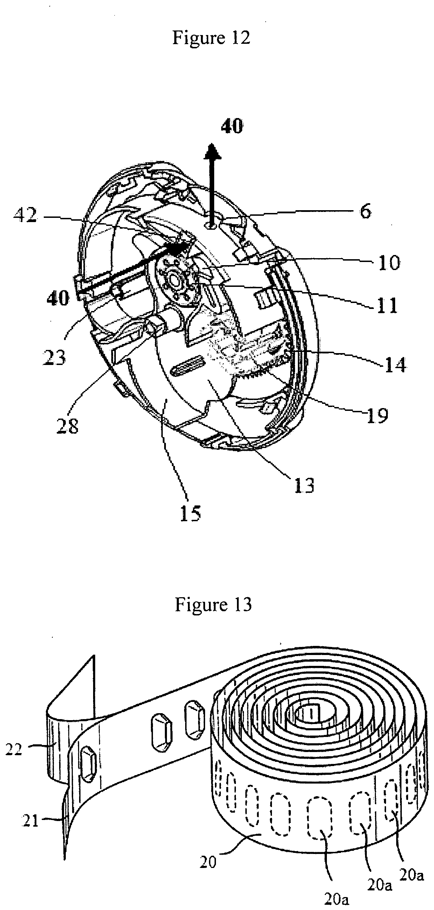

Upon sliding the mouthpiece cover (1) dispensing mechanism that is joined to mouthpiece cover (1) is actuated. Upon actuation of the dispensing mechanism blister strip (20) moves forward and upon peeling of the sheets of the blister strip a blister (20a) is open. Upon exact and accurate positioning of open blister (20a) the medicament in dry powder form becomes ready for inhalation at the dispensing station (42) of the inhaler device. Figure 12 shows track followed by airflow upon inhalation of the patient from the mouthpiece (5). Upon inhalation of the patient from mouthpiece (5), airflow that enters from airflow inlet (4) of the inhalation device, draws out the medicament in dry powder form that is present in dispensing station (42) and brings it to mouthpiece (5) and hence to the patient by passing through manifold (6) of the inhaler device.

Figure 13 shows the blister strip (20) in which the medicament in dry powder form is stored, carried and delivered to the patient through inhalation process, the base sheet (22) and the lid sheet (21). Additionally, it is possible to see the arrangement of the blisters (20a) on the peelable blister strip (20). Blister strip (20) is present in the inhaler in rolled form as shown in figure 13.

Active agent or active agents in dry powder form that are stored in the blister strip present in the dry powder inhaler of the present invention can be selected from a group comprising cromolyns, antiinfectives, antihistamines, anti-inflammatories, bronchodilators, leukotriene inhibitors, PDE IV inhibitors, antitussives, diuretics, anticholinergics, hormones, xanthines or combinations thereof. These active agents can be in the form of their salts solvates or esters.

Anticholinergic that are used as active agent can be selected from a group comprising : tiotropium or tiotropium bromide, oxitropium bromide, flutropyum bromide, ipratropium bromide, glicopyronium salts, trospium chloride, tolterodine, 2,2-diphenylpropionic acid, trophenol ester methobromide, 2,2-diphenylpropionic acid scopine ester metobromide, 2-fluoro-2,2-diphenyl acetic acid scopine ester metobromide, 2-fluoro-2,2-diphenyl acetic acid tropenol ester metobromide, 3,3',4,4'-tetrafluorobenzylic acid tropenol ester metobromide, 3,3',4,4'-tetrafluorobenzylic acid scopine ester metobromide, 4,4'-difluorobenzylic acid tropenol ester metobromide, 4,4'-difluorobenzylic acid scopine ester metobromide, 3,3'-difluorobenzylic acid tropenol ester metobromide, 3,3'-difluorobenzylic acid scopine ester metobromide, 9-hydroxy-fluoren-9-carboxylic acid tropenol ester metobromide, 9-fluoro-fluoren-9-carboxylic acid tropenol ester metobromide, 9-hydroxy-fluoren-9-carboxylic acid scopine ester metobromide, 9-fluoro-fluoren-9-carboxylic acid scopine ester metobromide, 9-methyl-fluoren-9-carboxylic acid tropenol ester metobromide, 9-methyl-fluoren-9-carboxylic acid scopine ester metobromide, benzylic acid cyclopropyltropin ester metobromide, 2,2-diphenylpropionic acid cyclopropyltropine ester metobromide, 9-hydroxy-fluorine-9-carboxylic acid cyclopropyltropin ester metobromide, 4,4'-difluorobenzylic acid methylester cyclopropyltropin ester metobromide, 9-hydroxy-xanthine-9-carboxylic acid tropenol ester metobromide, 9-hydroxy-xanthin-9-carboxylic acid scopine ester metobromide, 9-methyl-xanthine-9-carboxylic acid tropenol ester metobromide, 9-methyl-xanthin-9-carboxylic acid scopine ester metobromide, 9-ethyl-xanthine-9-carboxylic acid tropenol ester metobromide, 9-difluoromethyl-xantine-9-carboxylic acid tropenol ester metobromide and 9-hydroxymethyl-xanthine-9-carboxylic acid scopine ester metobromide; preferably their racemates, enantiomers or diastereomers, and solvates and/or hydrates.

Anti-inflammatory agents that are used as active agent are selected from a group comprising; prednisolone, prednison, buticocortpropionate, RPR-106541, flunisolid, beklomethasone, triamcinolon, budesonid, flutikazon, mometazon, ciclesonid, rofleponid, ST-126, dexametason, 6α, 9α -difluoro-17α-[(2-furanylcarbonyl)oxy]-11β-hydroxy-16α-methyl-3-oxo-androsta-1,4-diene-17β-carbothionic acid (S)-fluoromethylester, 6α,9α-difluoro-11β-hydroxy-16α-methyl-3-oxo-17α-propionyloxy-androsta-1,4-dien-17β-carbothionic acid (S)-(2-oxo-tetrahydro-furan-3S-yl)ester and ethyprednol-dichloroacetate (BNP-166); preferably racemates, enantiomera or diastereomers, and preferaby their acid addition salts, solvates and/or hydrates.

Leukotriene inhibitors that are used as active agent can be selected from a group comprising montelukast, 1-(((1(R)-3(3-(2-(2,3-dichlorothieno[3,2-b]pyridine-5-yl)-(E)-ethenyl)phenyl)-3-(2-(1-hydroxy-1-methylethyl)phenyl)propyl)thio)methyl)cyclopropane acetic acid, pranlukast, zafirlukast, [2-[[2-(4-tert-butyl-2-thiazolyl)-5-benzofuranyl]oxymethyl]phenyl]acetic acid, MCC-847 (ZD-3523), MN-001, MEN-91507 (LM-1507), VUF-5078, VUF-K-8707 and L-733321; preferably racemates, enantiomers or diastereomers, and preferably acid addition salts, solvates and/or hydrates.

PDE IV inhibitors that are used as active agent can be selected from a group comprising enprophilin, teophilin, roflumilast, ariflo (silomilast), CP-325,366, BY343, D-4396 (Sch-351591), AWD-12-281 (GW-842470), , CDP840, Bay-198004, D-4418, PD-168787, T-440, T-2585, arophilin, atizoram, V-11294A, C1-1018, CDC-801, CDC-3052, D-22888, YM-58997, Z-15370, and 9-cyclopentyl-5,6-dihydro-7-ethyl-3-(tert-butyl)-9H-pyrazolo[3,4-c]-1,2,4-triazolo[4,3-a]pyridin; preferably its racemates, enantiomers or diastereomers, and preferably their acid addition salts, solvates and/or hydrates.

Brochodilators used as active agents are preferably selected from salmeterol, salbutamol, formoterol, terbutaline, 3-(4-{[6-({(2fi)∼2-hydroxy-2-[4-hydroxy-3-(hydroxymethyl) phenyl]ethyl}amino)hexyl] oxy} butyl) benzenesulfonamide, 3-(3-{[7-({(2R)-2-hydroxy-2-[4-hydroxy-3-hydroxymethyl) phenyl] ethyl-amino) heptyl] oxy} propyl) benzenesulfonamide, and N-2{2-[4-(3-phenyl-4-methoxyphenyl)aminophenyl]ethyl}-2-hydroxy-2-(8-hydroxy-2(1H)-quilinolone-5-yl) ethylamine, 5-[(R)-2-(2-{4-[4-(2-amino-2-methyl-propoxy)-phenylamino]-phenyl}-ethylamino)-1-hydroxy-ethyl]-8-hydroxy-1H-quinoline-2-on, preferably their racemates, enantiomers or diastereomers or their acid addition salts, solvates and/or hydrates.

Antihistamines that are used as active agents are preferably selected from the group comprising amelexanose, astemizole azatidine, azelastine, acrivastin, brompheniramine, cetirizine, levocetirizine, efletirizine, chlorpheniramine, clemastine, cyclizin, carebastin, ciproheptadie, carbonoxamine, descarboathoxyloratadine, doxylamine, dimethidene, ebastine, epinastin, efletirizine, loratadin, levocabastine, mizolastine, mequitazine, mianserine, noberastine, meclizine, terfenadine, triphenylamine, temelastine, trimeprazine, triprolidine preferably cetirizine, levocetirizine, efletirizine and fexofenadine and pharmaceutcally acceptable salts, solvates, hydrates thereof.

The medicament formulation can be used for the treatment of many respiratory diseases such as asthma, chronic obstructive pulmonary disorder (COPD) and allergic rhinitis. Accordingly, the respiratory diseases include but are not restricted to allergic or non-allergic asthma in various phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition to this, the medicament composition is used especially for the symptomatic treatment of asthma, allergic rhinitis and COPD.

## Claims

1. A dry powder inhaler comprising:
- a mouthpiece (5) through which dry powder medicament is inhaled by the patient,
- a mouthpiece cover (1) for covering said mouthpiece (5),
- an elongate, peelable blister strip (20) containing the dry powder medicament,
- a dispensing mechanism which comprises gear mechanism of the dry powder inhaler and which is actuated by the movement of the mouthpiece cover (1) to open one blister (20a) of the blister strip (20) and making it ready for inhaling the dry powder medicament from said blister (20a),
- a stopper (2)
wherein before the inhalation of dry powder medicament, only a single movement is applied which is the movement of the mouthpiece cover (1) from one position in which the mouthpiece (5) is completely covered to another position in which:
- the mouthpiece (5) is completely uncovered,
- said stopper (2) which is in the exterior of the dry powder inhaler fixes the mouthpiece cover (1) preventing the reversible rotation of the peelable blister strip (20), and
- said dispensing mechanism is actuated for advancing and opening one blister (20a), making the medicament ready to be inhaled.

2. A dry powder inhaler according to claim 1, wherein said dispensing mechanism is actuated by the movement of the mouthpiece cover (1).

3. A dry powder inhaler according to any one of the preceding claims, wherein the dispensing mechanism is joined directly and mechanically to the mouthpiece cover (1).

4. A dry powder inhaler according to any one of the preceding claims, wherein all of the actions that are; the mouthpiece (5) is uncovered completely, the dispensing mechanism is actuated to advance the peelable blister strip (20) and dose counter (32) interacting with the dispensing mechanism counts doses left to be taken out of the dry powder inhaler, are achieved at the same time by the only movement of the mouthpiece cover (1).

5. A dry powder inhaler according to any one of the preceding claims, wherein the dispensing mechanism is placed in the housing (15) and the rotatable mouthpiece cover (1) is joined to this dispensing mechanism.

6. A dry powder inhaler according to any one of the preceding claims, wherein the mouthpiece cover (1) have a rotational movement.

7. A dry powder inhaler according to any one of the preceding claims, wherein the gear mechanism of the dispensing mechanism rotates irreversibly.

8. A dry powder inhaler according to any one of the preceding claims, wherein the movement of the mouthpiece cover (1) rotates central hub gear (28) which interacts with other gears of the dispensing mechanism

9. A dry powder inhaler according to claim 8, wherein the central hub gear (28) which is rotated by the movement of the mouthpiece cover (1), transmits the movement of the mouthpiece cover (1) to other gears of the gear mechanism

10. A dry powder inhaler according to claim 8 or claim 9, wherein the interaction between the central hub (28) and other gears of the dispensing mechanism provides for transmitting the movement of the mouthpiece cover (1) to other gears properly.

11. A dry powder inhaler according to any one of the preceding cliams, wherein the movement of the mouthpiece cover (1) from a position, in which the moutpiece is completely covered, to another position in which the mouthpiece (5) is completely uncovered, results in opening of one blister cavity (20a) for inhalation of the dry powder medicament in it.

## Patentansprüche

1. Ein Inhalationsapparat für Trockenpulver, das bestehend aus:
- einem Mundstück (5), wodurch Trockenpulvermedikament vom Patient eingeatmet wird,
- einem Mundstückdeckel (1) für Aufdeckung des besagten Mundstücks (5),
- einem länglichen, ablösbaren Blasenstreifen (20), der Trockenpulvermedikament beinhaltet,
- einem Verteilungsmechanismus, das aus Getrieben des Trockenpulvermedikaments besteht und durch Bewegung des Mundstückdeckels (1) betätigt wird, um eine Blase (20a) vom Blasenstreifen (20) zu eröffnen und ihn einzurichten, um Trockenpulvermedikament von besagter Blase (20a) einzuatmen,
- einem Verschluß (2),
worin vor Einatmung des Trockenpulvermedikaments nur eine einzige Bewegung gemacht wird, die sich auf den Vergang des Mundstückdeckels (1) von einer Position, wobei das Mundstück (5) völlig bedeckt ist, zur anderen Position bezieht, worin:
- das Mundstück (5) völlig unbedeckt ist,
- besagter Verschluß (2), der im Außen des Trockenpulverinhalators den Mundstückdeckel (1) fixiert, um eine umkehrbare Rotation des ablösbaren Blasenstreifens (20) zu verhindern, und
- besagter Verteilungsmechanismus für Vorangehen und Eröffnung einer Blase (20a) betätigt wird, um das Medikament für Einatmung einzurichten.

2. Ein Trockenpulverinhalator nach Anspruch 1, wobei der besagte Verteilungsmechanismus mit Bewegung des Mundstückdeckels (1) betätigt wird.

3. Ein Trockenpulverinhalator gemäß irgendwelcher der vorigen Forderungen, worin das Verteilungsmechanismus unmittelbar und mechanisch zum Mundstückdeckel (1) verbunden ist.

4. Ein Trockenpulverinhalator nach den vorhergehenden Ansprüche, wobei alle Tätigkeiten wie folgendes sind; das Mundstück (5) ist völlig unbedeckt, der Verteilungsmechanismus ist betätigt, um den ablösbaren Blasenstreifen (20) und Dosenzähler (32) voranzugehen, der mit aufeinander wirkenden Verteilungsmechanismus die vom Trockenpulverinhalator aufzunehmende Dosenzahl einzählt, wobei diese gleichzeitig nur mit einziger Bewegung des Mundstückdeckels (1) vollgebracht werden.

5. Ein Trockenpulverinhalator nach den vorhergehenden Ansprüche, wobei der Verteilungsmechanismus in das Gehäuse (15) hingelegt und der drehbare Mundstück-deckel (1) zu diesem Mechanismus verbunden wird.

6. Ein Trockenpulverinhalator nach den vorhergehenden Ansprüche, wobei der Mundstückdeckel (1) eine Drehbewegung hat.

7. Ein Trockenpulverinhalator nach den vorhergehenden Ansprüche, wobei der Getriebemechanismus des Verteilungsmechanismus irreversibel rotiert.

8. Ein Trockenpulverinhalator nach den vorhergehenden Ansprüche, wobei die Bewegung des Mundstückdeckels (1) das zentrale Nabengetriebe (28) rotiert, das mit anderen Getrieben des Verteilungsmechanismus bewirkt.

9. Ein Trockenpulvermechanismus nach Anspruch 8, wobei das zentrale Nabengetriebe (28), das mit Bewegung des Mundstückdeckels (1) rotiert wird, die Bewegung des Mundstückdeckels (1) zu anderen Getrieben des Getriebemechanismus überträgt.

10. Ein Trockenpulvermechanismus nach Anspruch 8 oder 9, wobei die Wechselwirkung zwischen dem Nabengetriebe (28) und anderen Getrieben des Verteilungsmechanismus die Bewegung des Mundstückdeckels (1) zu anderen Getrieben geeignet sicherstellt.

11. Ein Trockenpulverinhalator nach den vorhergehenden Ansprüche, wo die Bewegung des Mundstückdeckels (1) von einer Position, wobei das Mundstück völlig bedeckt ist, zu einer anderen Position, wobei das Mundstück (5) völlig unbedeckt wird, zur Eröffnung einer Blasenkavität (20a) für Einatmung vom Trockenpulvermedikament darin ergibt.

## Revendications

1. Un inhalateur à poudre sèche comprenant:
- un embout (5) à travers lequel le médicament en poudre sèche est inhalé par le patient,
- un couvercle d'embout (1) pour recouvrir ledit embout (5),
- une plaquette alvéolée allongée, pelable (20) contenant le médicament en poudre sèche,
- un mécanisme de distribution qui comprend un mécanisme d'engrenage de l'inhalateur à poudre sèche et qui est actionné par le mouvement du couvercle de l'embout (1) pour ouvrir une plaquette (20a) de la plaquette alvéolée (20) et la préparer pour inhaler le medicament en poudre sèche de ladite plaquette (20a),
- un bouchon (2)
dans lequel, avant inhalation d'un médicament en poudre sèche, seul un mouvement unique est appliqué qui est le mouvement du couvercle de l'embout (1) d'une position dans laquelle l'embout (5) est complètement recouvert à une autre position dans laquelle:
- l'embout (5) est totalement découvert,
- ledit bouchon (2) qui est à l'extérieur de l'inhalateur à poudre sèche, fixe le couvercle de l'embout (1) empêchant la rotation réversible de la plaquette alvéolée pelable (20) et
- ledit mécanisme de distribution est actionné pour faire avancer et ouvrir une plaquette (20a), ce qui rend le médicament prêt à être inhalé.

2. Un inhalateur à poudre sèche selon la revendication 1, dans lequel ledit mécanisme de distribution est actionné par le mouvement du couvercle de l'embout (1).

3. Un inhalateur à poudre sèche selon l'une quelconque des revendications précédentes, dans lequel le mécanisme de distribution est relié directement et mécaniquement au couvercle de l'embout (1).

4. Un inhalateur à poudre sèche selon l'une quelconque des revendications précédentes, dans lequel toutes les actions qui sont; l'embout (5) est totalement découverte, le mécanisme de distribution est actionné pour faire avancer la plaquette alvéolée pelable (20) et le compteur de dose (32) interagissant avec le mécanisme de distribution compte les doses laissées à retirer de l'inhalateur à poudre sèche sont atteintes au même moment par le seul mouvement du couvercle de l'embout (1).

5. Un inhalateur à poudre sèche selon l'une quelconque des revendications précédentes, dans lequel le mécanisme de distribution est placé dans le boîtier (15) et le couvercle rotatif (1) de l'embout est relié à ce mécanisme de distribution.

6. Un inhalateur à poudre sèche selon l'une quelconque des revendications précédentes, dans lequel le couvercle de l'embout (1) présente un mouvement de rotation.

7. Un inhalateur à poudre sèche selon l'une quelconque des revendications précédentes, dans lequel le mécanisme d'engrenage du mécanisme de distribution tourne de manière irréversible.

8. Un inhalateur à poudre sèche selon l'une quelconque des revendications précédentes, dans lequel le mouvement du couvercle de l'embout (1) fait tourner l'engrenage du moyeu central (28) qui interagit avec d'autres engrenages du mécanisme de distribution

9. Un inhalateur à poudre sèche selon la revendication 8, dans lequel l'engrenage de moyeu central (28) qui tourne par le mouvement du couvercle de l'embout (1) transmet le mouvement du couvercle de l'embout (1) à d'autres engrenages du mécanisme d'engrenage

10. Un inhalateur à poudre sèche selon la revendication 8 ou la revendication 9, dans lequel l'interaction entre le moyeu central (28) et d'autres engrenages du mécanisme de distribution permet de transmettre de manière appropriée le mouvement du couvercle de l'embout (1) à d'autres engrenages.

11. Un inhalateur à poudre sèche selon l'une quelconque des revendications précédentes, dans lequel le mouvement du couvercle de l'embout (1) d'une position, dans lequel l'embout est complètement recouvert, à une autre position dans laquelle l'embout (5) est complètement découvert, a pour consequence l'ouverture d'une cavité de la plaquette (20a) pour l'inhalation du médicament en poudre sèche.
